(19) Europäisches Patentamt
European Patent Office
Office européen des brevets

(11) **EP 4 454 564 B1**

(12) **EUROPEAN PATENT SPECIFICATION**

(45) Date of publication and mention
of the grant of the patent:
**30.04.2025  Bulletin 2025/18**

(51) International Patent Classification (IPC):
**A61B 5/352** (2021.01)        **A61B 5/366** (2021.01)
**A61B 5/00** (2006.01)

(21) Application number: **23180201.8**

(22) Date of filing: **20.06.2023**

(52) Cooperative Patent Classification (CPC):
**A61B 5/352; A61B 5/366; A61B 5/725;** A61B 5/308

(54) **METHOD OF AUTOMATIC QRS COMPLEX DETECTION IN ELECTROCARDIOGRAM SIGNAL**

VERFAHREN ZUR AUTOMATISCHEN QRS-KOMPLEX-DETEKTION IN EINEM
ELEKTROKARDIOGRAMMSIGNAL

PROCÉDÉ DE DÉTECTION AUTOMATIQUE DE COMPLEXE QRS DANS UN SIGNAL
D'ÉLECTROCARDIOGRAMME

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority:  **28.04.2023  PL 44466823**

(43) Date of publication of application:
**30.10.2024  Bulletin 2024/44**

(73) Proprietor: **Akademia Gorniczo-Hutnicza im.
Stanislawa
Staszica w Krakowie
30-059 Krakow (PL)**

(72) Inventors:
• **Szaflarski, Andrzej**
  **Szaflary 34-424 (PL)**
• **Reklewski, Wojciech**
  **02-493 Warszawa (PL)**
• **Heryan, Katarzyna**
  **30-109 Kraków (PL)**
• **Kisiel-Dorohinicki, Marek**
  **Kraków 31-463 (PL)**
• **Miskowicz, Marek**
  **31-621 Kraków (PL)**

(74) Representative: **Wlasienko, Jozef et al
Polservice
Kancelaria Rzeczników
Patentowych Sp. z o.o.
Ul. Bluszczanska 73
00-712 Warszawa (PL)**

(56) References cited:
**EP-A1- 3 970 615**

• **GUTIERREZ-RIVAS RAQUEL ET AL: "Novel Real-
Time Low-Complexity QRS Complex Detector
Based on Adaptive Thresholding", IEEE
SENSORS JOURNAL, IEEE, USA, vol. 15, no. 10,
1 October 2015 (2015-10-01), pages 6036 - 6043,
XP011666999, ISSN: 1530-437X, [retrieved on
20150818], DOI: 10.1109/JSEN.2015.2450773**
• **JAIN SHWETA ET AL: "QRS detection using
adaptive filters: A comparative study", ISA
TRANSACTIONS, INSTRUMENT SOCIETY OF
AMERICA. PITTSBURGH, US, vol. 66, 10 October
2016 (2016-10-10), pages 362 - 375,
XP029903602, ISSN: 0019-0578, DOI: 10.1016/
J.ISATRA.2016.09.023**
• **XIAO RUPING ET AL: "Ultra-low power QRS
detection using adaptive thresholding based on
forward search interval technique", 2017
INTERNATIONAL CONFERENCE ON ELECTRON
DEVICES AND SOLID-STATE CIRCUITS (EDSSC),
IEEE, 18 October 2017 (2017-10-18), pages 1 - 2,
XP033274171, DOI: 10.1109/
EDSSC.2017.8126486**

**Description**

The subject of the invention is a method of automatic QRS complex detection in an electrocardiogram signal ECG applicable in biomedical diagnostics.

State of the art

Electrocardiography is one of the most commonly performed diagnostic procedures that allows the analysis and assessment of the heart's electrical activity. Reading the potential difference between two electrodes placed on the body of a patient, mostly on the chest, allows plotting the so-called electrocardiographic curve (ECG curve) representing the course of the mentioned potential difference over time. Evaluation of the ECG curve requires analysis to be made by a doctor or a specialist in order to detect possible irregularities of the heart activity of the examined person. In order to support the interpretation and evaluation of the ECG curve, many devices and algorithms have been developed that allow to at least partially automate the ECG curve analysis, increase its reliability and accelerate the diagnosis.

The ECG curve is cyclical due to the repetitive activity of the heart. In each single cycle of the ECG curve, there are characteristic waveform features corresponding to cardiac activity, which most often include the P-wave, the QRS complex and the T-wave. The P-wave corresponds to depolarization of the atrial muscle, the QRS complex corresponds to depolarization of the ventricular muscle, and the T-wave corresponds to ventricular repolarization. The description of the heart's activity and the interpretation of the ECG curve is the subject of numerous studies available in the art.

In order to enable an automated analysis of the ECG curve, it is essential to reliably detect the characteristic features of the ECG curve, in particular the above-mentioned QRS complex.

From the publication Gutiérrez-Rivas, Raquel, et al. "Novel real-time low-complexity QRS complex detector based on adaptive thresholding." IEEE Sensors Journal, vol. 15, no. 10, s. 6036-6043, 2015 a QRS complex detector based on adaptive thresholding is known.

From the application P.435394 a method of detecting a QRS complex in an electrocardiogram (ECG) signal is known, in which an ABS_DIFF_SHORT signal generated on an output of an ABS_DIFF_SHORT_MODULE module, which is the difference of the current instantaneous value of the ECG signal provided by an ECG_MODULE measuring module, and the mean value of a SHORT_AVG of the ECG signal calculated for a time interval T_SHORT, are monitored using the COMP comparator. Then, the COMP comparator detects the moment when an ABS_DIFF_SHORT signal reaches a threshold TH value previously determined with a TH_MODULE module, and then, when the ABS_DIFF_SHORT signal reaches the threshold TH value, the countdown of a SEARCHING_WINDOW time window starts with a PULSE_GENERATOR pulse generator which has fixed-length for search for a R wave in the QRS complex of the ECG signal. In the next step, using a PEAK_DETECTOR detection module, the maximum value of the ABS_DIFF_LONG_MODULE module produced at its output is recorded during the SEARCHING_WINDOW time window of the ABS_DIFF_LONG signal, which is the difference of the current instantaneous value of the ECG signal and the average LONG_AVG value of the ECG signal calculated for a segment of a T_LONG time segment while the PEAK_DETECTOR detection module the moment of the detected maximum value of the ABS_DIFF_LONG signal is recorded during the SEARCHING_WINDOW time window. After that, the maximum value of the ABS_DIFF_LONG signal detected by the PEAK_DETECTOR detection module is stored by means of a R_AMPLITUDE_MEMORY memory module, which is considered to be the R-wave amplitude in the QRS complex.

From the application P.435396 a device for detecting the QRS complex of electrocardiogram signal is known.

The object of the present invention is to provide an improved method of automatic QRS complex detection in an electrocardiogram signal with increased reliability of R-wave detection. This object is solved by the method according to claim 1.

According to the present invention, a method of automatic QRS complex detection in an electrocardiogram signal consists in receiving an ECG signal obtained by means of a measuring module ECG MODULE monitoring electrical heart activity of a patient, wherein the ECG signal is represented in the form of samples provided by the measuring module ECG MODULE at equal time intervals measured by a clock and successively stored in a buffer BUF by means of a CLK signal generated in the measuring module ECG MODULE, and then in that

a) by means of a module TH MODULE an initial value of a threshold TH_PREPROCESSED is set, and then

b) a signal Y_PREPROCESSED generated at an output of a module PREPROCESSING MODULE is monitored by means of a comparator COMPARATOR , which signal is obtained by generating by means of the module PREPROCESSING MODULE a signal $Y0$ being the difference between a value Y of the considered sample of the ECG signal and a sample value of the ECG signal previously stored in the buffer BUF, which has been received a predetermined number $Nd$ of samples earlier, and determining by means of the module PREPROCESSING MODULE a signal $Y1$ being the sum of the considered sample of the signal $Y0$ and a value of a predetermined number N-1 of samples of the signal *Y0* immediately preceding the considered sample of the signal *Y0*, and dividing this sum by a number N and squaring such obtained number,

c) by means of the module TH MODULE the value of the threshold TH_PREPROCESSED is systematically decreased every sampling interval of the ECG signal starting from its initial value previously determined by means of the module TH MODULE by multiplying the current threshold value TH_PREPROCESSED, in time instants of receiving successive samples of the ECG signal, by a predetermined and less than one constant value TH_REDUCING_FACTOR and these operations are repeated the required number of times until a time instant of reaching the threshold TH_PREPROCESSED by the signal Y_PREPROCESSED is detected by means of the comparator COMPARATOR,

d) when the time instant of reaching the current value of the threshold TH_PREPROCESSED by the signal Y_PREPROCESSED is detected, a time window SEARCHING_WINDOW is started to be measured by means of a pulse generator SEARCHING WINDOW PULSE GENERATOR for searching for the R-wave in the QRS complex of the ECG signal,

e) next, during the time window SEARCHING_WINDOW a maximum value of the signal Y_PREPROCESSED is detected by means of a detection module PEAK DETECTOR and this value is stored in a module PEAK AMPLITUDE MEMORY.

The method according to the invention is characterized in that

in step a) an initial value for a threshold TH_FACTOR is set by means of a module TH FACTOR MODULE,

in step c) local extremes of the ECG signal are searched for by means of a module EXTREMA MODULE,

after detecting by means of the module EXTREMA MODULE that the considered sample of the ECG signal is the local extreme of the ECG signal, a value of a parameter FACTOR is determined for this sample of the ECG signal by means of a module FACTOR MODULE, which parameter specifies how distinct local extreme the considered sample of the ECG signal is against the neighbouring samples of the ECG signal, and for this purpose the parameter FACTOR is assigned a value the bigger, the greater the amplitude of the considered local extreme represented by the considered sample of the ECG signal than the values of the neighbouring samples of the ECG signal,

next, after determining the value of the parameter FACTOR for the considered sample of the ECG signal being the local extreme of the ECG signal, the determined value of the parameter FACTOR is compared with the threshold TH_FACTOR previously determined by means of the module TH FACTOR MODULE, and if the value of the parameter FACTOR is greater than the set threshold TH_FACTOR, the value of the parameter FACTOR and the time instant of receiving the considered sample of the ECG signal previously registered by means of the module EXTREMA MODULE, for which time instant the parameter FACTOR has been determined, are stored in a list THRESHOLD_WINDOW_LIST in a memory of a module FACTOR MEMORY,

wherein these operations are repeated the required number of times until a time instant of reaching the threshold TH_PREPROCESSED by the signal Y_PREPROCESSED is detected by means of the comparator COMPARATOR, whose value is systematically decreased every sampling interval of the ECG signal by successive multiplying the value of the threshold TH_PREPROCESSED, in time instants of receiving successive samples of the ECG signal, by the predetermined and less than one constant value TH_REDUCING_FACTOR,

wherein in step d) the time window SEARCHING_WINDOW has a determined minimal length SEARCHING_WINDOW_LEN_MIN,

in step e) during the time window SEARCHING_WINDOW local extremes of the ECG signal are simultaneously determined by means of the module EXTREMA MODULE,

next, for each one of the samples recognized as a local extreme it is determined, by determining a value of the parameter FACTOR by means of the module FACTOR MODULE, how distinct local extreme the considered sample of the ECG signal is against the neighbouring samples,

for each detected local extreme the value of the parameter FACTOR and a time instant of receiving the considered sample of the ECG signal previously registered by means of the module EXTREMA MODULE, for which time instant the parameter FACTOR has been determined, are stored in a list SEARCHING_WINDOW_LIST in the memory module FACTOR MEMORY,

after a set minimum duration SEARCHING_WINDOW_LEN_MIN of the time window SEARCHING_WINDOW the measurement of the time window SEARCHING_WINDOW is terminated by the pulse generator SEARCHING WINDOW PULSE GENERATOR, provided that the maximum value of the signal Y_PREPROCESSED registered during the time window SEARCHING_WINDOW and reduced by the current value of the signal Y_PREPROCESSED reaches a predetermined threshold MIN_DIFF, what is detected by a module PULSE EXTENSION,

after the end of the time window SEARCHING_WINDOW such a value FACTOR_MAX1 of the parameter FACTOR is selected from the list SEARCHING_WINDOW_LIST by means of a module FACTOR SELECTOR, which is the greatest among those stored in the list SEARCHING_WINDOW_LIST and the maximum value FACTOR_MAX1 of the parameter FACTOR considered to be the double amplitude of the R-wave in the QRS complex is stored in a module AMPLITUDE MEMORY, and then the maximum value FACTOR_MAX1 of the parameter FACTOR is copied to an output memory module OUTPUT MEMORY,

at the same time a time instant of occurrence of the maximum value FACTOR_MAX1 of the parameter FACTOR is stored in a module TIMESTAMP MEMORY, which is also recognized as the time instant of occurrence of the R-wave in the QRS complex, and then the time instant of occurrence of the maximum value FACTOR_MAX1 of the parameter FACTOR is copied to the output memory module OUTPUT MEMORY,

next, from the list THRESHOLD_WINDOW_LIST in the module FACTOR MEMORY those values of the parameter FACTOR stored in this list are removed by means of the module FACTOR SELECTOR, whose time instant of occurrence precedes by less than a set time period R-TOR_MIN since the time instant of occurrence of the R-wave in the QRS complex stored in the output memory module OUTPUT MEMORY,

next, provided that the number of remaining elements in the list THRESHOLD_WINDOW_LIST is not greater than a set constant MAX_ABOVE_TH, that value FACTOR_MAX2 of the parameter FACTOR is selected from the list THRESHOLD_WINDOW_LIST by means of a module FACTOR SELECTOR, which is the greatest among those stored in the list THRESHOLD_WINDOW_LIST and the maximum value FACTOR_MAX2 of the parameter FACTOR considered to be double the amplitude of the previous R-wave in the QRS complex, than the R-wave previously detected for the value FACTOR_MAX1 of the parameter FACTOR, and is stored in the module AMPLITUDE MEMORY, and then the maximum value FACTOR_MAX2 of the parameter FACTOR is copied to the output memory module OUTPUT MEMORY,

at the same time a time instant of occurrence of the maximum value FACTOR_MAX2 of the parameter FACTOR is stored in the module TIMESTAMP MEMORY, which is also recognized as the time instant of occurrence of the previous R-wave in the QRS complex, than the R-wave previously detected for the value FACTOR_MAX1 of the parameter FACTOR, and then the time instant of occurrence of the maximum value FACTOR_MAX2 of the parameter FACTOR is copied to the output memory module OUTPUT MEMORY,

next, the sum value of the values of the parameters FACTOR_MAX1 obtained so far is updated by means of the module TH FACTOR MODULE by adding the current value FACTOR_MAX1 to the previous sum value and a counter of performed algorithm work cycles is updated and on this basis an arithmetic mean of the values FACTOR found so far within time windows is calculated,

and then the parameter TH_FACTOR is assigned that value among the pair MIN_TH_FACTOR and NEW_TH_FACTOR, which is not less than the second value in the pair MIN_TH_FACTOR and NEW_TH_FACTOR, wherein NEW_TH_FACTOR is the product of a parameter EXPECTED_TH_FACTOR and of a weighted mean with weights, respectively, (1 - TH_FACTOR_LAST_PEAK_COEF) and TH_FACTOR_LAST_PEAK_COEF of the arithmetic mean of values of the parameter FACTOR for the R-waves in the QRS complex found so far and the value of the parameter FACTOR_MAX1 for the last found R-wave,

next, the sum value of the maximum values of the signal Y_PREPROCESSED obtained so far is updated by means of the module TH MODULE by adding the current maximum value Y_PREPROCESSED to the previous sum value and the counter of performed algorithm work cycles is updated and on this basis an arithmetic mean of the values Y_PREPROCESSED found so far within time windows is calculated,

next, the parameter TH_PREPROCESSED is assigned a new value, which is a weighted mean with weights, respectively, (1 - TH_PREPROCESSED_LAST_PEAK_COEF) and TH_PREPROCESSED_LAST_PEAK_COEF of the arithmetic mean of maximum values of the signal Y_PREPROCESSED found so far within time windows for detecting R-waves in the QRS complex and of the maximum value of the signal Y_PREPROCESSED for the current time window SEARCHING_WINDOW, in which the last R-wave has been identified,

next, by means of a module REFRACTORY WINDOW PULSE GENERATOR a measurement of a time window REFRACTORY_WINDOW is started, the end of which occurs after time REFRACTORY_LEN since the time instant of detecting the last R-wave,

next, during REFRACTORY_WINDOW all the stored elements on the lists THRESHOLD_WINDOW_LIST and SEARCHING_WINDOW_LIST are removed from the module FACTOR MEMORY in order to prepare the module for the next work cycle,

after terminating the measurement of the time window REFRACTORY_WINDOW, monitoring of the signal Y_PREPROCESSED generated at the output of the module PREPROCESSING MODULE by means of the comparator COMPARATOR is resumed, and then the cycle is repeated the desired number of times.

In an advantageous embodiment of the method according to the invention, the local extremes of the ECG signal are searched for by means of the module EXTREMA MODULE in such a way that

-    after receiving the considered sample of the ECG signal by means of the measuring module ECG MODULE and storing this sample of the ECG signal by means of the module BUF, a maximum value LOCAL_EXT_MAX_LEFT and a minimum value LOCAL_EXT_MIN_LEFT are determined by means of the EXTREMA MODULE for a set of predetermined number LOCAL_EXT_LEN of samples of the ECG signal stored in the buffer BUF that immediately precede the considered sample and of the considered sample of the ECG signal, and then the maximum value LOCAL_EXT_MAX_LEFT and the minimum value LOCAL_EXT_MIN_LEFT are stored by means of the module EXTREMA MODULE, and also

-    after receiving by means of the measuring module ECG_MODULE and collecting in the buffer BUF the predetermined number LOCAL_EXT_LEN of samples of the ECG signal that follow immediately after the considered sample of the ECG signal, a maximum value LOCAL_EXT_MAX_RIGHT and a minimum value LOCAL_EXT_MIN_RIGHT are determined by means of the module EXTREMA MODULE for a set of predetermined number LOCAL_EXT_LEN of samples of the ECG signal and of the considered sample of the ECG signal, and then these maximum value LOCAL_EXT_MAX_RIGHT and minimum value LOCAL_EXT_MIN_RIGHT are stored by means of the module EXTREMA MODULE,

next, the previously determined maximum value LOCAL_EXT_MAX_LEFT and the previously determined minimum value LOCAL_EXT_MIN_LEFT are successively compared by means of the module EXTREMA MODULE, for the predetermined number LOCAL_EXT_LEN of samples of the ECG signal immediately preceding the considered sample of the ECG signal and stored in the buffer BUF, with the value Y of the considered sample of the ECG signal,

and also the previously determined maximum value LOCAL_EXT_MAX_RIGHT and the previously determined LOCAL_EXT_MIN_RIGHT are successively compared by means of the module EXTREMA MODULE, for the predetermined number LOCAL_EXT_LEN of samples of the ECG signal that follow immediately after the considered sample of the ECG signal, with the value Y of the considered sample of the ECG signal,

and if the result of the comparisons shows equality with the value Y of the considered sample of the ECG signal for at least one of the maximum values LOCAL_EXT_MAX_LEFT and LOCAL_EXT_MAX_ RIGHT or the minimum values LOCAL_EXT_MIN_LEFT and LOCAL_EXT_MIN_RIGHT obtained previously for the groups of samples immediately preceding and immediately following the considered sample of the ECG signal, then the considered sample of the ECG signal is recognized as a local extreme.

In another advantageous embodiment of the method according to the invention, the value of the parameter FACTOR, which specifies how distinct local extreme the considered sample of the ECG signal is against the neighbouring samples of the ECG signal, is determined by means of the module FACTOR MODULE in such a way that

a minimum value MIN_LEFT and a maximum value MAX_LEFT of samples of the ECG signal are determined for a predetermined number MAX_WIDTH of samples of the ECG signal that immediately precede the considered sample and are stored in the buffer BUF, and then, this maximum value MAX_LEFT and this minimum value MIN_LEFT are stored by means of the module FACTOR MODULE for the predetermined number MAX_WIDTH of samples of the ECG signal that immediately precede the considered sample, and also

- after receiving by means of the measuring module ECG MODULE and collecting in the buffer BUF the predetermined number MAX_WIDTH of samples of the ECG signal that follow immediately after the considered sample of the ECG signal, a maximum value MAX_RIGHT and a minimum value MIN_RIGHT are determined by means of the module FACTOR MODULE for the predetermined number MAX_WIDTH of samples of the ECG signal, and then the maximum value MAX_RIGHT and the minimum value MIN_RIGHT are stored by means of the module FACTOR MODULE for the predetermined number MAX_WIDTH of samples of the ECG signal that follow immediately after the considered sample of signal,

and the value of the parameter FACTOR is determined by taking as the parameter FACTOR the value of that one among the pair of the parameters FACTOR_MAX and FACTOR_MIN, which is not less than the second one, where the values of the parameters FACTOR_MAX and FACTOR_MIN are determined according to the formulas:

$$FACTOR\_MAX = (Y - MIN\_LEFT) + (Y - MIN\_RIGHT)$$

$$FACTOR\_MIN = (MAX\_LEFT - Y) + (MAX\_RIGHT - Y)$$

wherein Y is the value of the considered sample of the ECG signal, for which the factor FACTOR is determined.

The method according to the invention provides increased reliability of detecting R-waves even in the presence of cardiac anomalies and in the presence of interference or distortion of the ECG signal. The features of the method furthermore make it possible to keep the computational complexity low, whereby the method can be implemented in portable devices, e.g. by implementing in the form of computer program instructions on a storage medium.

The subject of the invention in embodiments is presented in the drawing, in which:

Fig. 1 is a simplified diagram of an electrocardiogram signal waveform with marked P- and T-waves and QRS complex;

Fig. 2 is a block diagram of a device for automatic QRS complex detection in an electrocardiogram signal for implementing the method according to the present invention.

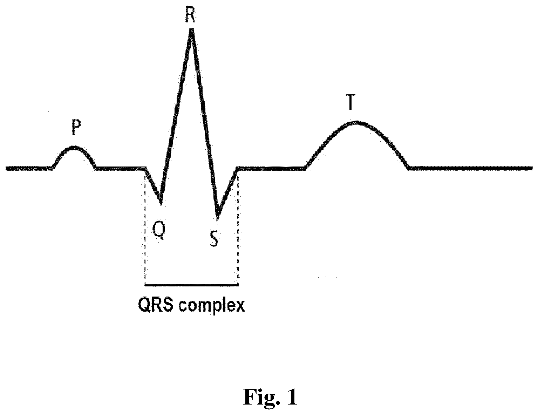

Fig. 1 is a simplified diagram of an electrocardiogram signal waveform (ECG) with marked P- and T- waves and QRS complex. Such electrocardiogram signal (ECG) is received by means of a measuring module through electrodes placed on the body of a patient. The ECG signal is characterized by a sequence of positive and negative deviations (so-called waves) from the isoelectric line, which correspond to periods of time in which no cardiac excitation is found. The group of the largest waves includes a negative deflection (Q-wave), a positive deflection (R-wave) and again a negative deflection (S-wave) and is referred to as the so-called QRS complex. The greatest amplitude in the QRS complex usually has the R-wave, so that the detection of the QRS complex often comes down to detection of the R-wave. The statistics of time intervals between R-waves and of amplitudes of the R-waves is an important diagnostic information used in medicine, among others, to identify abnormalities in the heart activity.

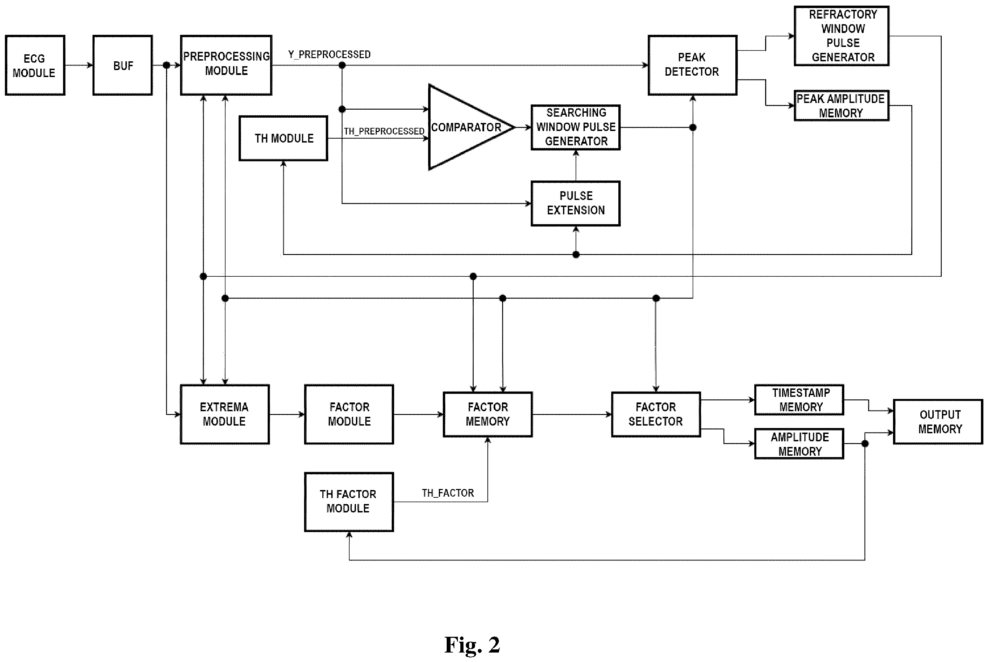

Fig. 2 is a block diagram of a device for automatic QRS complex detection in an electrocardiogram signal for implementing the method according to the present invention. As shown in the figure, the exemplary device for implementing the method according to the present invention comprises a number of mutually connected modules, which are used to implement particular steps of the method according to the invention. According to the presented block diagram, an output of a module ECG MODULE is connected to an input of a buffer module BUF, whose output is connected to an input of a module PREPROCESSING MODULE. Outputs of the module PREPROCESSING MODULE and of a module TH MODULE are connected to inputs of a comparator COMPARATOR, whose output is connected to a module SEARCHING WINDOW PULSE GENERATOR. Inputs of a module PEAK DETECTOR are connected to outputs of the modules PREPROCESSING MODULE and SEARCHING WINDOW PULSE GENERATOR, respectively, whereas its outputs are connected to inputs of modules REFRACTORY WINDOW PULSE GENERATOR and PEAK AMPLITUDE MEMORY, respectively. Inputs of a module PULSE EXTENSION are connected to outputs of the modules PROCESSING MODULE and PEAK AMPLITUDE MEMORY, respectively, whereas its output is connected to an input of the module SEARCHING WINDOW PULSE GENERATOR. Inputs of a module EXTREMA MODULE are connected to inputs of the modules BUF, REFRACTORY WINDOW PULSE GENERATOR, SEARCHING WINDOW PULSE GENERATOR, respectively, whereas its output is connected to an input of a module FACTOR MODULE. Particular inputs of a module FACTOR MEMORY are connected to outputs of modules REFRACTORY WINDOW PULSE GENERATOR, SEARCHING WINDOW PULSE GENERATOR and TH FACTOR MODULE, respectively. A module FACTOR SELECTOR has two inputs connected to the modules FACTOR MEMORY and SEARCHING WINDOW PULSE GENERATOR, respectively, and two outputs connected to modules TIMESTAMP MEMORY and AMPLITUDE MEMORY, whose outputs are connected to inputs of a module OUTPUT MEMORY. The output of the module AMPLITUDE MEMORY is also connected to an input of the module TH FACTOR MODULE. Functions of the individual modules and related steps of the method according to the invention are explained in the following detailed description of an embodiment.

The measuring module ECG MODULE provides an electrocardiogram signal (ECG) in the form of signal samples, which are delivered at a given frequency determined by a signal CLK, which frequency in the embodiment is 360Hz. By means of the module TH MODULE an initial value of a threshold TH_PREPROCESSED is set, equal to a maximum value of a signal Y_PREPROCESSED detected within first 3 seconds of the ECG signal obtained by means of the measuring module ECG MODULE, and an initial value of a threshold TH_FACTOR is set by means of the module TH FACTOR MODULE, equal to a parameter MIN_TH_FACTOR = 1.22 [mV]. The full range of possible changes of the analogue ECG signal is +5mV. The resolution of the A/D converter in this embodiment is $2^{11}=2048$, and therefore 1.22 mV corresponds to 244 "steps" of the A/D converter. The resolution of the A/D converter is 5μV. Next, a signal Y_PREPROCESSED generated at the output of the module PREPROCESSING MODULE is monitored by means of the comparator COMPARATOR, obtained by:

generating by means of the module PREPROCESSING MODULE a signal $Y0$ being the difference between a value Y of the considered sample of the ECG signal and a sample value of the ECG signal previously stored in the buffer BUF, which has been received a predetermined number $Nd$ = 7 samples earlier (for signal CLK=360Hz, which corresponds to time 19.4445 ms),

and determining by means of the module PREPROCESSING MODULE a signal $Y1$ being the sum of the value $Y0$ of the considered sample of the signal $Y0$ and a value of a predetermined number of 7 samples (N-1=7; N=8) of the signal $Y0$ immediately preceding the considered sample of the signal $Y0$ (for signal CLK=360Hz), which corresponds to time 22.2222 ms, and dividing this sum by a number N=8 (CLK=360Hz) and squaring such obtained number.

At the same time, by means of the module TH MODULE the value of the threshold TH_PREPROCESSED is systematically decreased every sampling interval of the ECG signal (1/360 s for signal CLK =360Hz) starting from its initial value previously determined by means of the module TH MODULE by multiplying the current threshold value TH_PREPROCESSED, in time instants of receiving successive samples of the ECG signal, by a predetermined and less than one constant value TH_REDUCING_FACTOR = 0.9818 (value for CLK=360Hz).

At the same time, local extremes of the ECG signal are searched for by means of the module EXTREMA MODULE.

After detecting by means of the module EXTREMA MODULE that the considered sample of the ECG signal is the local extreme of the ECG signal, a value of a parameter FACTOR is determined for this sample of the ECG signal by means of the module FACTOR MODULE, which parameter specifies how distinct local extreme the considered sample of the ECG signal is against the neighbouring samples of the ECG signal, and for this purpose the parameter FACTOR is assigned a value the bigger, the greater the amplitude of the considered local extreme represented by the considered sample of the ECG signal than the values of the neighbouring samples of the ECG signal.

Next, after determining the value of the parameter FACTOR for the considered sample of the ECG signal being the local extreme of the ECG signal, the determined value of the parameter FACTOR is compared with the threshold TH_FACTOR previously determined by means of the module TH FACTOR MODULE, and if the value of the parameter FACTOR is greater than the set threshold TH_FACTOR, the value of the parameter FACTOR and the time instant of receiving the considered sample of the ECG signal previously registered by means of the module EXTREMA MODULE, for which time instant the parameter FACTOR has been determined, are stored in a list THRESHOLD_WINDOW_LIST in a memory of the module FACTOR MEMORY, wherein these operations are repeated the required number of times until a time instant of reaching the threshold TH_PREPROCESSED by the signal Y_PREPROCESSED is detected by means of the comparator COMPARATOR, whose value is systematically decreased every sampling interval of the ECG signal (1/360 s) by successive multiplying the value of the threshold TH_PREPROCESSED, in time instants of receiving successive samples of the ECG signal, by a predetermined and less than one constant value TH_REDUCING_FACTOR = 0.9818 (value for 360Hz).

When the time instant of reaching the current value of the threshold TH_PREPROCESSED by the signal Y_PREPROCESSED is detected, a time window SEARCHING_WINDOW is started to be measured by means of the pulse generator SEARCHING WINDOW PULSE GENERATOR for searching for the R-wave in the QRS complex of the ECG signal having a determined minimal length SEARCHING_WINDOW_LEN_MIN = 260ms (which corresponds to duration of 93 samples).

Next, during the time window SEARCHING_WINDOW a maximum value of the signal Y_PREPROCESSED is detected by means of the detection module PEAK DETECTOR and this value is stored in the module PEAK AMPLITUDE MEMORY.

At the same time, during the time window SEARCHING_WINDOW local extremes of the ECG signal are determined by means of the module EXTREMA MODULE, and next, for each one of the samples recognized as a local extreme it is determined, by determining a value of the parameter FACTOR by means of the module FACTOR MODULE, how distinct local extreme the considered sample of the ECG signal is against the neighbouring samples.

For each detected local extreme the value of the parameter FACTOR and a time instant of receiving the considered sample of the ECG signal previously registered by means of the module EXTREMA MODULE, for which time instant the parameter FACTOR has been determined, are stored in a list SEARCHING_WINDOW_LIST in the memory module FACTOR MEMORY.

After a set minimum duration SEARCHING_WINDOW_LEN_MIN = 260ms of the time window SEARCHING_WINDOW the measurement of the time window SEARCHING_WINDOW is terminated by the pulse generator SEARCHING WINDOW PULSE GENERATOR, provided that the maximum value of the signal Y_PREPROCESSED registered during the time window SEARCHING_WINDOW and reduced by the current value of the signal Y_PREPROCESSED reaches a predetermined threshold MIN_DIFF = 0.062 [mV], what is detected by the module PULSE EXTENSION.

After the end of the time window SEARCHING_WINDOW such a value FACTOR_MAX1 of the parameter FACTOR is selected from the list SEARCHING_WINDOW_LIST by means of a module FACTOR SELECTOR, which is the greatest among those stored in the list SEARCHING_WINDOW_LIST and the maximum value FACTOR_MAX1 of the parameter FACTOR considered to be the double amplitude of the R-wave in the QRS complex is stored in a module AMPLITUDE MEMORY, and next, the maximum value FACTOR_MAX1 of the parameter FACTOR is copied to the output memory module OUTPUT MEMORY.

At the same time a time instant of occurrence of the maximum value FACTOR_MAX1 of the parameter FACTOR is stored in the module TIMESTAMP MEMORY, which is also recognized as the time instant of occurrence of the R-wave in the QRS complex, and then the time instant of occurrence of the maximum value FACTOR_MAX1 of the parameter FACTOR is copied to the output memory module OUTPUT MEMORY.

Next, from the list THRESHOLD_WINDOW_LIST in the module FACTOR MEMORY those maximum values of the parameter FACTOR stored in this list are removed by means of the module FACTOR SELECTOR, whose time instant of occurrence precedes by less than a set time period R-TO-R_MIN = 200 ms (which corresponds to 72 samples at signal frequency CLK = 360Hz) since the time instant of occurrence of the R-wave in the QRS complex stored in the output memory module OUTPUT MEMORY.

Next, provided that the number of remaining elements in the list THRESHOLD_WINDOW_LIST is not greater than a set constant MAX_ABOVE_TH = 2, that value FACTOR_MAX2 of the parameter FACTOR is selected from the list THRESHOLD_WINDOW_LIST by means of the module FACTOR SELECTOR, which is the greatest among those stored in the list THRESHOLD_WINDOW_LIST and the maximum value FACTOR_MAX2 of the parameter FACTOR considered to be double the amplitude of the previous R-wave in the QRS complex than the R-wave previously detected for the value FACTOR_MAX1 of the parameter FACTOR, and is stored in the module AMPLITUDE MEMORY, , and then the maximum value FACTOR_MAX2 of the parameter FACTOR is copied to the output memory module OUTPUT MEMORY.

At the same time a time instant of occurrence of the maximum value FACTOR_MAX2 of the parameter FACTOR is stored in the module TIMESTAMP MEMORY, which is also recognized as the time instant of occurrence of the previous R-wave in the QRS complex, than the R-wave previously detected for the value FACTOR_MAX1 of the parameter FACTOR, and then the time instant of occurrence of the maximum value FACTOR_MAX2 of the parameter FACTOR is copied to the output memory module OUTPUT MEMORY.

Next, the sum value of the values of the parameters FACTOR_MAX1 obtained so far is updated by means of the module TH FACTOR MODULE by adding the current value FACTOR_MAX1 to the previous sum value and a counter of performed algorithm work cycles is updated and on this basis an arithmetic mean of the values FACTOR found so far within time windows is calculated, and then the parameter TH_FACTOR is assigned that value among the pair MIN_TH_FACTOR = 1.22 [mV] and NEW_TH_FACTOR, which is not less than the second value in the pair MIN_TH_FACTOR and NEW_TH_FACTOR, wherein NEW_TH_FACTOR is the product of a parameter EXPECTED_TH_FACTOR = 0.48 and of a weighted mean with weights, respectively, (1 - TH_FACTOR_LAST_PEAK_COEF) = 0.6 and TH_FACTOR_LAST_PEAK_COEF = 0.4 of the arithmetic mean of values of the parameter FACTOR for the R-waves in the QRS complex found so far and the value of the parameter FACTOR_MAX1 for the last found R-wave.

Next, the sum value of the maximum values of the signal Y_PREPROCESSED obtained so far is updated by means of the module TH MODULE by adding the current maximum value Y_PREPROCESSED to the previous sum value and the counter of performed algorithm work cycles is updated and on this basis an arithmetic mean of the values Y_PREPROCESSED found so far within time windows is calculated, and next, the parameter TH_PREPROCESSED is assigned a new value, which is a weighted mean with weights, respectively, (1 - TH_PREPROCESSED_LAST_PEAK_COEF) = 0.3 and TH_PREPROCESSED_LAST_PEAK_COEF = 0.7 of the arithmetic mean of maximum values of the signal Y_PREPROCESSED found so far within time windows for detecting R-waves in the QRS complex and of the maximum value of the signal Y_PREPROCESSED for the current time window SEARCHING_WINDOW, in which the last R-wave has been identified.

Next, by means of the module REFRACTORY WINDOW PULSE GENERATOR a measurement of a time window REFRACTORY_WINDOW is started, the end of which occurs after time REFRACTORY_LEN = 200ms since the time instant of detecting the last R-wave.

Next, during REFRACTORY_WINDOW all the stored elements on the lists THRESHOLD_WINDOW_LIST and SEARCHING_WINDOW_LIST are removed from the module FACTOR MEMORY in order to prepare the module for the next work cycle.

After terminating the measurement of the time window REFRACTORY_WINDOW, monitoring of the signal Y_PREPROCESSED generated at the output of the module PREPROCESSING MODULE by means of the comparator COMPARATOR is resumed, and then the cycle is repeated the desired number of times.

In an advantageous embodiment of the method according to the invention the local extremes of the ECG signal are searched for by means of the module EXTREMA MODULE in such a way that

- after receiving the considered sample of the ECG signal by means of the measuring module ECG MODULE and storing this sample of the ECG signal by means of the module BUF, a maximum value LOCAL_EXT_MAX_LEFT and a minimum value LOCAL_EXT_MIN_LEFT are determined by means of the EXTREMA MODULE for a set of predetermined number LOCAL_EXT_LEN = 6 samples (which corresponds to time 0.017 ms at signal frequency CLK = 360 Hz) of the ECG signal stored in the buffer BUF that immediately precede the considered sample and of the considered sample of the ECG signal, and then the maximum value LOCAL_EXT_MAX_LEFT and the minimum value LOCAL_EXT_MIN_LEFT are stored by means of the module EXTREMA MODULE, and also

- after receiving by means of the measuring module ECG_MODULE and collecting in the buffer BUF the predetermined number LOCAL_EXT_LEN = 6 samples of the ECG signal that follow immediately after the considered sample of the ECG signal, a maximum value LOCAL_EXT_MAX_RIGHT and a minimum value LOCAL_EXT_MIN_RIGHT are determined by means of the module EXTREMA MODULE for a set of predetermined number LOCAL_EXT_LEN of samples of the ECG signal and of the considered sample of the ECG signal, and then these maximum value LOCAL_EXT_MAX_RIGHT and minimum value LOCAL_EXT_MIN_RIGHT are stored by means of the module EXTREMA MODULE.

Next, the previously determined maximum value LOCAL_EXT_MAX_LEFT and the previously determined minimum value LOCAL_EXT_MIN_LEFT are successively compared by means of the module EXTREMA MODULE, for the predetermined number LOCAL_EXT_LEN of samples of the ECG signal immediately preceding the considered sample of the ECG signal and stored in the buffer BUF, with the value Y of the considered sample of the ECG signal, and also the previously determined maximum value LOCAL_EXT_MAX_ RIGHT and the previously determined LOCAL_EXT_MIN_RIGHT are successively compared by means of the module EXTREMA MODULE, for the predetermined number LOCAL_EXT_LEN = 6 samples of the ECG signal that follow immediately after the considered sample of the ECG signal, with the value Y of the considered sample of the ECG signal. If the result of the comparisons shows equality with the value Y of the considered sample of the ECG signal for at least one of the maximum values LOCAL_EXT_MAX_LEFT and LOCAL_EXT_MAX_ RIGHT or the minimum values LOCAL_EXT_MIN_LEFT and LOCAL_EXT_MIN_RIGHT obtained previously for the groups of samples immediately preceding and immediately following the considered sample of the ECG signal, then the considered sample of the ECG signal is recognized as a local extreme.

In another advantageous embodiment of the method according to the invention, the value of the parameter FACTOR, which specifies how distinct local extreme the considered sample of the ECG signal is against the neighbouring samples of the ECG signal, is determined by means of the module FACTOR MODULE in such a way that

a minimum value MIN_LEFT and a maximum value MAX_LEFT of samples of the ECG signal are determined for a predetermined number MAX_WIDTH = 17 samples (which corresponds to 0,047ms for signal CLK=360Hz) of the ECG signal that immediately precede the considered sample and are stored in the buffer BUF, and then, this maximum value MAX_LEFT and this minimum value MIN_LEFT are stored by means of the module FACTOR MODULE for the predetermined number MAX_WIDTH = 17 samples of the ECG signal that immediately precede the considered sample, and also

- after receiving by means of the measuring module ECG MODULE and collecting in the buffer BUF the predetermined number MAX_WIDTH = 17 samples (which corresponds to 0,047ms for signal CLK=360Hz) of the ECG signal that follow immediately after the considered sample of the ECG signal, a maximum value MAX_RIGHT and a minimum value MIN_RIGHT are determined by means of the module FACTOR MODULE for the predetermined number MAX_WIDTH of samples of the ECG signal, and then the maximum value MAX_RIGHT and the minimum value MIN_RIGHT are stored by means of the module FACTOR MODULE for the predetermined number MAX_WIDTH = 17 samples of the ECG signal that follow immediately after the considered sample of signal, and the value of the parameter FACTOR is determined by taking as the parameter FACTOR the value of that one among the pair of the parameters FACTOR_MAX and FACTOR_MIN, which is not less than the second one, where the values of the parameters FACTOR_MAX and FACTOR_MIN are determined according to the formulas:

$$FACTOR\_MAX = (Y - MIN\_LEFT) + (Y - MIN\_RIGHT)$$

$$FACTOR\_MIN = (MAX\_LEFT - Y) + (MAX\_RIGHT - Y)$$

wherein Y is the value of the considered sample of the ECG signal, for which the factor FACTOR is determined.

## Claims

1. A method of QRS complex detection in an electrocardiogram signal (ECG) consisting in receiving the ECG signal obtained by means of a measuring module ECG MODULE monitoring electrical heart activity of a patient, wherein the ECG signal is represented in the form of samples provided by the measuring module ECG MODULE at equal time intervals measured by a clock and successively stored in a buffer BUF by means of a CLK signal generated in the measuring module ECG MODULE, and then in that

   a) by means of a module TH MODULE an initial value of a threshold TH_PREPROCESSED is set, and then
   b) a signal Y_PREPROCESSED generated at an output of a module PREPROCESSING MODULE is monitored by means of a comparator COMPARATOR, which signal is obtained by

   generating by means of the module PREPROCESSING MODULE a signal $Y0$ being the difference between a value Y of the considered sample of the ECG signal and a sample value of the ECG signal previously stored in the buffer BUF, which has been received a predetermined number $Nd$ of samples earlier,
   and determining by means of the module PREPROCESSING MODULE a signal $Y1$ being the sum of the considered sample of the signal $Y0$ and a value of a predetermined number N-1 of samples of the signal $Y0$ immediately preceding the considered sample of the signal $Y0$, and dividing this sum by a number N and squaring such obtained number,

   c) by means of the module TH MODULE the value of the threshold TH_PREPROCESSED is systematically decreased every sampling interval of the ECG signal starting from its initial value previously determined by means of the module TH MODULE by multiplying the current threshold value TH_PREPROCESSED, in time instants of receiving successive samples of the ECG signal, by a predetermined and less than one constant value TH_REDUCING_FACTOR and these operations are repeated the required number of times until a time instant of reaching the threshold TH_PREPROCESSED by the signal Y_PREPROCESSED is detected by means of the comparator COMPARATOR,
   d) when the time instant of reaching the current value of the threshold TH_PREPROCESSED by the signal Y_PREPROCESSED is detected, a time window SEARCHING_WINDOW is started to be measured by means of a pulse generator SEARCHING WINDOW PULSE GENERATOR for searching for the R-wave in the QRS complex of the ECG signal,
   e) next, during the time window SEARCHING_WINDOW a maximum value of the signal Y_PREPROCESSED is detected by means of a detection module PEAK DETECTOR and this value is stored in a module PEAK AMPLITUDE MEMORY,
   **characterized in that**

   in step a) an initial value for a threshold TH_FACTOR is set by means of a module TH FACTOR MODULE,
   in step c) local extremes of the ECG signal are searched for by means of a module EXTREMA MODULE,
   after detecting by means of the module EXTREMA MODULE that the considered sample of the ECG signal is the local extreme of the ECG signal, a value of a parameter FACTOR is determined for this sample of the ECG signal by means of a module FACTOR MODULE, which parameter specifies how distinct local extreme the considered sample of the ECG signal is against the neighbouring samples of the ECG signal, and for this purpose the parameter FACTOR is assigned a value the bigger, the greater the amplitude of the considered local extreme represented by the considered sample of the ECG signal than the values of the neighbouring samples of the ECG signal,

next, after determining the value of the parameter FACTOR for the considered sample of the ECG signal being the local extreme of the ECG signal, the determined value of the parameter FACTOR is compared with the threshold TH_FACTOR previously determined by means of the module TH FACTOR MODULE, and if the value of the parameter FACTOR is greater than the set threshold TH_FACTOR, the value of the parameter FACTOR and the time instant of receiving the considered sample of the ECG signal previously registered by means of the module EXTREMA MODULE, for which time instant the parameter FACTOR has been determined, are stored in a list THRESHOLD_WINDOW_LIST in a memory of a module FACTOR MEMORY, wherein these operations are repeated the required number of times until a time instant of reaching the threshold TH_PREPROCESSED by the signal Y_PREPROCESSED is detected by means of the comparator COMPARATOR, whose value is systematically decreased every sampling interval of the ECG signal by successive multiplying the value of the threshold TH_PREPROCESSED, in time instants of receiving successive samples of the ECG signal, by the predetermined and less than one constant value TH_REDUCING_FACTOR,

wherein in step d) the time window SEARCHING_WINDOW has a determined minimal length SEARCHING_WINDOW_LEN_MIN,

in step e) during the time window SEARCHING_WINDOW local extremes of the ECG signal are simultaneously determined by means of the module EXTREMA MODULE,

next, for each one of the samples recognized as a local extreme it is determined, by determining a value of the parameter FACTOR by means of the module FACTOR MODULE, how distinct local extreme the considered sample of the ECG signal is against the neighbouring samples,

for each detected local extreme the value of the parameter FACTOR and a time instant of receiving the considered sample of the ECG signal previously registered by means of the module EXTREMA MODULE, for which time instant the parameter FACTOR has been determined, are stored in a list SEARCHING_WINDOW_LIST in the memory module FACTOR MEMORY,

after a set minimum duration SEARCHING_WINDOW_LEN_MIN of the time window SEARCHING_WINDOW the measurement of the time window SEARCHING_WINDOW is terminated by the pulse generator SEARCHING WINDOW PULSE GENERATOR, provided that the maximum value of the signal Y_PREPROCESSED registered during the time window SEARCHING_WINDOW and reduced by the current value of the signal Y_PREPROCESSED reaches a predetermined threshold MIN_DIFF, what is detected by a module PULSE EXTENSION,

after the end of the time window SEARCHING_WINDOW such a value FACTOR_MAX1 of the parameter FACTOR is selected from the list SEARCHING_WINDOW_LIST by means of a module FACTOR SELECTOR, which is the greatest among those stored in the list SEARCHING_WINDOW_LIST and the maximum value FACTOR_MAX1 of the parameter FACTOR considered to be double the amplitude of the R-wave in the QRS complex is stored in a module AMPLITUDE MEMORY, and then the maximum value FACTOR_MAX1 of the parameter FACTOR is copied to an output memory module OUTPUT MEMORY,

at the same time a time instant of occurrence of the maximum value FACTOR_MAX1 of the parameter FACTOR is stored in a module TIMESTAMP MEMORY, which is also recognized as the time instant of occurrence of the R-wave in the QRS complex, and then the time instant of occurrence of the maximum value FACTOR_MAX1 of the parameter FACTOR is copied to the output memory module OUTPUT MEMORY,

next, from the list THRESHOLD_WINDOW_LIST in the module FACTOR MEMORY those values of the parameter FACTOR stored in this list are removed by means of the module FACTOR SELECTOR, whose time instant of occurrence precedes by less than a set time period R-TOR_MIN since the time instant of occurrence of the R-wave in the QRS complex stored in the output memory module OUTPUT MEMORY,

next, provided that the number of remaining elements in the list THRESHOLD_WINDOW_LIST is not greater than a set constant MAX_ABOVE_TH, that value FACTOR_MAX2 of the parameter FACTOR is selected from the list THRESHOLD_WINDOW_LIST by means of a module FACTOR SELECTOR, which is the greatest among those stored in the list THRESHOLD_WINDOW_LIST and the maximum value FACTOR_MAX2 of the parameter FACTOR considered to be double the amplitude of the previous R-wave in the QRS complex is stored in the module AMPLITUDE MEMORY, rather than the R-wave previously detected for the value FACTOR_MAX1 of the parameter FACTOR, and then the maximum value FACTOR_MAX2 of the parameter FACTOR is copied to the output memory module OUTPUT MEMORY,

at the same time a time instant of occurrence of the maximum value FACTOR_MAX2 of the parameter FACTOR is stored in the module TIMESTAMP MEMORY, which is also recognized as the time instant of occurrence of the previous R-wave in the QRS complex, rather than the R-wave previously detected for the value FACTOR_MAX1 of the parameter FACTOR, and then the time instant of occurrence of the maximum value FACTOR_MAX2 of the parameter FACTOR is copied to the output memory module OUTPUT MEMORY,

next, the sum value of the values of the parameters FACTOR_MAX1 obtained so far is updated by means of the module TH FACTOR MODULE by adding the current value FACTOR_MAX1 to the previous sum value and a counter of performed algorithm work cycles is updated and on this basis an arithmetic mean of the values FACTOR found so far within time windows is calculated,

and then the parameter TH_FACTOR is assigned that value among the pair MIN_TH_FACTOR and NEW_TH_FACTOR, which is not less than the second value in the pair MIN_TH_FACTOR and NEW_TH_FACTOR, wherein NEW_TH_FACTOR is the product of a parameter EXPECTED_TH_FACTOR and of a weighted mean with weights, respectively, (1 - TH_FACTOR_LAST_PEAK_COEF) and TH_FACTOR_LAST_PEAK_COEF of the arithmetic mean of values of the parameter FACTOR for the R-waves in the QRS complex found so far and the value of the parameter FACTOR_MAX1 for the last found R-wave,

next, the sum value of the maximum values of the signal Y_PREPROCESSED obtained so far is updated by means of the module TH MODULE by adding the current maximum value Y_PREPROCESSED to the previous sum value and the counter of performed algorithm work cycles is updated and on this basis an arithmetic mean of the values Y_PREPROCESSED found so far within time windows is calculated,

next, the parameter TH_PREPROCESSED is assigned a new value, which is a weighted mean with weights, respectively, (1 - TH_PREPROCESSED_LAST_PEAK_COEF) and TH_PREPROCESSED_LAST_PEAK_COEF of the arithmetic mean of maximum values of the signal Y_PREPROCESSED found so far within time windows for detecting R-waves in the QRS complex and of the maximum value of the signal Y_PREPROCESSED for the current time window SEARCHING_WINDOW, in which the last R-wave has been identified,

next, by means of a module REFRACTORY WINDOW PULSE GENERATOR a measurement of a time window REFRACTORY_WINDOW is started, the end of which occurs after time REFRACTORY_LEN since the time instant of detecting the last R-wave,

next, during REFRACTORY_WINDOW all the stored elements on the lists THRESHOLD_WINDOW_LIST and SEARCHING_WINDOW_LIST are removed from the module FACTOR MEMORY in order to prepare the module for the next work cycle,

after terminating the measurement of the time window REFRACTORY_WINDOW, monitoring of the signal Y_PREPROCESSED generated at the output of the module PREPROCESSING MODULE by means of the comparator COMPARATOR is resumed, and then the cycle is repeated the desired number of times.

2. The method according to claim 1 **characterized in that** the local extremes of the ECG signal are searched for by means of the module EXTREMA MODULE in such a way that

- after receiving the considered sample of the ECG signal by means of the measuring module ECG MODULE and storing this sample of the ECG signal by means of the module BUF, a maximum value LOCAL_EXT_MAX_LEFT and a minimum value LOCAL_EXT_MIN_LEFT are determined by means of the EXTREMA MODULE for a set of predetermined number LOCAL_EXT_LEN of samples of the ECG signal stored in the buffer BUF that immediately precede the considered sample and of the considered sample of the ECG signal, and then the maximum value LOCAL_EXT_MAX_LEFT and the minimum value LOCAL_EXT_MIN_LEFT are stored by means of the module EXTREMA MODULE, and also

- after receiving by means of the measuring module ECG_MODULE and collecting in the buffer BUF the predetermined number LOCAL_EXT_LEN of samples of the ECG signal that follow immediately after the considered sample of the ECG signal, a maximum value LOCAL_EXT_MAX_RIGHT and a minimum value LOCAL_EXT_MIN_RIGHT are determined by means of the module EXTREMA MODULE for a set of predetermined number LOCAL_EXT_LEN of samples of the ECG signal and of the considered sample of the ECG signal, and then these maximum value LOCAL_EXT_MAX_RIGHT and minimum value LOCAL_EXT_MIN_RIGHT are stored by means of the module EXTREMA MODULE,

next, the previously determined maximum value LOCAL_EXT_MAX_LEFT and the previously determined minimum value LOCAL_EXT_MIN_LEFT are successively compared by means of the module EXTREMA MODULE, for the predetermined number LOCAL_EXT_LEN of samples of the ECG signal immediately preceding the considered sample of the ECG signal and stored in the buffer BUF, with the value Y of the considered sample of the ECG signal,

and also the previously determined maximum value LOCAL_EXT_MAX_RIGHT and the previously determined LOCAL_EXT_MIN_RIGHT are successively compared by means of the module EXTREMA MODULE, for the predetermined number LOCAL_EXT_LEN of samples of the ECG signal that follow immediately after the considered sample of the ECG signal, with the value Y of the considered sample of the ECG signal, and if the result of the comparisons shows equality with the value Y of the considered sample of the ECG signal for at least one of the maximum values LOCAL_EXT_MAX_LEFT and LOCAL_EXT_MAX_ RIGHT or the minimum values LOCAL_EXT_MIN_LEFT and LOCAL_EXT_MIN_RIGHT obtained previously for the groups of samples immediately preceding and immediately following the considered sample of the ECG signal, then the considered sample of the ECG signal is recognized as a local extreme.

3. The method according to claim 1 **characterized in that** the value of the parameter FACTOR, which specifies how distinct local extreme the considered sample of the ECG signal is against the neighbouring samples of the ECG signal, is determined by means of the module FACTOR MODULE in such a way that

a minimum value MIN_LEFT and a maximum value MAX_LEFT of samples of the ECG signal are determined for a predetermined number MAX_WIDTH of samples of the ECG signal that immediately precede the considered sample and are stored in the buffer BUF, and then, this maximum value MAX_LEFT and this minimum value MIN_LEFT are stored by means of the module FACTOR MODULE for the predetermined number MAX_WIDTH of samples of the ECG signal that immediately precede the considered sample, and also

- after receiving by means of the measuring module ECG MODULE and collecting in the buffer BUF the predetermined number MAX_WIDTH of samples of the ECG signal that follow immediately after the considered sample of the ECG signal, a maximum value MAX_RIGHT and a minimum value MIN_RIGHT are determined by means of the module FACTOR MODULE for the predetermined number MAX_WIDTH of samples of the ECG signal, and then the maximum value MAX_RIGHT and the minimum value MIN_RIGHT are stored by means of the module FACTOR MODULE for the predetermined number MAX_WIDTH of samples of the ECG signal that follow immediately after the considered sample of signal,

and the value of the parameter FACTOR is determined by taking as the parameter FACTOR the value of that one among the pair of the parameters FACTOR_MAX and FACTOR_MIN, which is not less than the second one, where the values of the parameters FACTOR_MAX and FACTOR_MIN are determined according to the formulas:

$$FACTOR\_MAX = (Y - MIN\_LEFT) + (Y - MIN\_RIGHT)$$

$$FACTOR\_MIN = (MAX\_LEFT - Y) + (MAX\_RIGHT - Y)$$

wherein Y is the value of the considered sample of the ECG signal, for which the factor FACTOR is determined.

4. A storage medium comprising computer executable instructions for implementing the method according to claims 1 - 3.

**Patentansprüche**

1. Ein Verfahren zur Erkennung des QRS-Komplexes in einem Elektrokardiogrammsignal (EKG), das darin besteht, das EKG-Signal zu empfangen, das mittels eines Messmoduls ECG MODULE erhalten wurde, das die elektrische Herzaktivität eines Patienten überwacht, wobei das EKG-Signal in Form von Proben dargestellt wird, die vom Messmodul ECG MODULE in gleichen Zeitintervallen bereitgestellt werden, die von einer Uhr gemessen und nacheinander in einem Puffer BUF mittels eines im Messmodul ECG MODULE erzeugten CLK-Signals gespeichert werden, und dann dadurch, dass

a) mittels eines Moduls TH MODULE ein Anfangswert eines Schwellenwerts TH_PREPROCESSED festgelegt wird, und dann
b) ein an einem Ausgang eines Moduls PREPROCESSING MODULE erzeugtes Signal Y_PREPROCESSED mittels eines Komparators COMPARATOR überwacht wird, wobei dieses Signal erhalten wird, indem

mittels des Moduls PREPROCESSING MODULE ein Signal *Y0* erzeugt wird, das die Differenz zwischen einem Wert Y der betrachteten Probe des EKG-Signals und einem zuvor im Puffer BUF gespeicherten Probenwert des EKG-Signals ist, das eine vorgegebene Anzahl *Nd* von Proben zuvor empfangen wurde, und Bestimmen eines Signals *Y1* mittels des Moduls PREPROCESSING MODULE, das die Summe der betrachteten Probe des Signals *Y0* und eines Werts einer vorgegebenen Anzahl N-1 von Proben des Signals *Y0* ist, die der betrachteten Probe des Signals *Y0* unmittelbar vorausgehen, und Teilen dieser Summe durch eine Zahl N und Quadrieren dieser erhaltenen Zahl,

c) mittels des Moduls TH MODULE der Wert des Schwellenwerts TH_PREPROCESSED in jedem Abtastintervall des EKG-Signals systematisch verringert wird, ausgehend von seinem Anfangswert, der zuvor mittels des Moduls TH MODULE bestimmt wurde, indem der aktuelle Schwellenwert TH_PREPROCESSED in Zeitpunkten des Empfangs aufeinanderfolgender Proben des EKG-Signals mit einem vorgegebenen und kleiner als eins konstanten Wert TH_REDUCING_FACTOR multipliziert wird, und diese Vorgänge das erforderliche Vielfache wiederholt werden, bis ein Zeitpunkt des Erreichens des Schwellenwerts TH_PREPROCESSED durch das Signal Y_PREPROCESSED mittels des Komparators COMPARATOR erkannt wird,
d) wenn der Zeitpunkt des Erreichens des aktuellen Wertes des Schwellenwerts TH_PREPROCESSED durch das Signal Y_PREPROCESSED erkannt wird, ein Zeitfenster SEARCHING_WINDOW gestartet wird, das mittels eines Impulsgenerators SEARCHING WINDOW PULSE GENERATOR zum Suchen nach der R-Welle im QRS-Komplex des EKG-Signals gemessen wird,
e) anschließend während des Zeitfensters SEARCHING_WINDOW ein Maximalwert des Signals Y_PREPROCESSED mittels eines Detektionsmoduls PEAK DETECTOR erkannt wird und dieser Wert in einem Modul PEAK AMPLITUDE MEMORY gespeichert wird,
**dadurch gekennzeichnet, dass**

in Schritt a) mittels eines Moduls TH FACTOR MODULE ein Anfangswert für einen Schwellenwert TH_FACTOR gesetzt wird,
in Schritt c) mittels eines Moduls EXTREMA MODULE nach lokalen Extrema des EKG-Signals gesucht wird, nachdem mittels des Moduls EXTREMA MODULE detektiert wurde, dass die betrachtete Probe des EKG-Signals das lokale Extrema des EKG-Signals ist, mittels eines Moduls FACTOR MODULE ein Wert eines Parameters FACTOR für diese Probe des EKG-Signals bestimmt wird, der angibt, wie deutlich sich das lokale Extrema der betrachteten Probe des EKG-Signals von den benachbarten Proben des EKG-Signals unterscheidet, zu diesem Zweck dem Parameter FAKTOR ein umso größerer Wert zugewiesen wird, je größer die Amplitude des betrachteten lokalen Extremums ist, das durch die betrachtete Probe des EKG-Signals dargestellt wird, als die Werte der benachbarten Proben des EKG-Signals,
als nächstes, nachdem der Wert des Parameters FACTOR für die betrachtete Probe des EKG-Signals bestimmt wurde, das das lokale Extremum des EKG-Signals ist, der bestimmte Wert des Parameters FACTOR mit dem zuvor mittels des Moduls TH FACTOR MODULE bestimmten Schwellenwert TH_FACTOR verglichen wird, und wenn der Wert des Parameters FACTOR größer als der festgelegte Schwellenwert TH_FACTOR ist, der Wert des Parameters FACTOR und der Zeitpunkt des Empfangs der betrachteten Probe des EKG-Signals, die zuvor mittels des Moduls EXTREMA MODULE registriert wurde, für den der Parameter FACTOR bestimmt wurde, in einer Liste THRESHOLD_WINDOW_LIST in einem Speicher eines Moduls FACTOR MEMORY gespeichert werden,
wobei diese Vorgänge das erforderliche Vielfache wiederholt werden, bis ein Zeitpunkt des Erreichens des Schwellenwerts TH_PREPROCESSED durch das Signal Y_PREPROCESSED mittels des Komparators COMPARATOR erkannt wird, dessen Wert in jedem Abtastintervall des EKG-Signals systematisch verringert wird, indem der Wert des Schwellenwerts TH_PREPROCESSED in Zeitpunkten des Empfangs aufeinanderfolgender Proben des EKG-Signals sukzessive mit dem vorbestimmten und kleiner als eins konstanten Wert TH_REDUCING_FACTOR multipliziert wird,
wobei in Schritt d) das Zeitfenster SEARCHING_WINDOW eine bestimmte minimale Länge SEARCHING_WINDOW_LEN_MIN hat,
in Schritt e) während des Zeitfensters SEARCHING_WINDOW gleichzeitig lokale Extrema des EKG-Signals mittels des Moduls EXTREMA MODULE bestimmt werden,
dann für jede der als lokale Extrema erkannten Proben durch Bestimmen eines Werts des Parameters FACTOR mittels des Moduls FACTOR MODULE bestimmt wird, wie deutlich sich das lokale Extrem der betrachteten Probe des EKG-Signals von den benachbarten Proben unterscheidet,
für jede erkannte lokale Extrema der Wert des Parameters FACTOR und ein Zeitpunkt des Empfangs der betrachteten Probe des EKG-Signals zuvor mittels des Moduls EXTREMA MODULE registriert werden, für welchen Zeitpunkt der Parameter FACTOR ermittelt wurde, in einer Liste SEARCHING_WINDOW_LIST im Speichermodul FACTOR MEMORY gespeichert werden,

nach einer eingestellten Mindestdauer SEARCHING_WINDOW_LEN_MIN des Zeitfensters SEARCHING_WINDOW die Messung des Zeitfensters SEARCHING_WINDOW durch den Impulsgenerator SEARCHING WINDOW PULSE GENERATOR beendet wird, sofern der während des Zeitfensters SEARCHING_WINDOW registrierte und um den aktuellen Wert des Signals Y_PREPROCESSED reduzierte Maximalwert des Signals Y_PREPROCESSED einen vorgegebenen Schwellenwert MIN_DIFF erreicht, was durch ein Modul PULSE EXTENSION erkannt wird,

nach dem Ende des Zeitfensters SEARCHING_WINDOW mittels eines Moduls FACTOR SELECTOR derjenige Wert FACTOR_MAX1 des Parameters FACTOR aus der Liste SEARCHING_WINDOW_LIST ausgewählt wird, der der größte unter den in der Liste gespeicherten SEARCHING_WINDOW_LIST und der Maximalwert FACTOR_MAX1 des Parameters FACTOR ist, der als die doppelte Amplitude der R-Welle im QRS-Komplex betrachtet wird, in einem Modul AMPLITUDE MEMORY gespeichert werden, und dann der Maximalwert FACTOR_MAX1 des Parameters FACTOR in ein Ausgabespeichermodul OUTPUT MEMORY kopiert wird,

gleichzeitig ein Zeitpunkt des Auftretens des Maximalwerts FACTOR_MAX1 des Parameters FACTOR in einem Modul TIMESTAMP MEMORY gespeichert wird, der auch als Zeitpunkt des Auftretens der R-Welle im QRS-Komplex erkannt wird, und dann der Zeitpunkt des Auftretens des Maximalwerts FACTOR_MAX1 des Parameters FACTOR in das Ausgabespeichermodul OUTPUT MEMORY kopiert wird,

als nächstes aus der Liste THRESHOLD_WINDOW_LIST im Modul FACTOR MEMORY diejenigen Werte des in dieser Liste gespeicherten Parameters FACTOR mittels des Moduls FACTOR SELECTOR entfernt werden, deren Zeitpunkt des Auftretens um weniger als eine festgelegte Zeitspanne R-TO-R_MIN vor dem Zeitpunkt des Auftretens der R-Welle im QRS-Komplex liegt, der im Ausgabespeichermodul OUTPUT MEMORY gespeichert ist,

als nächstes, vorausgesetzt dass die Anzahl der verbleibenden Elemente in der Liste THRESHOLD_WINDOW_LIST nicht größer als eine festgelegte Konstante MAX_ABOVE_TH ist, mittels eines Moduls FACTOR SELECTOR jener Wert FACTOR_MAX2 des Parameters FACTOR aus der Liste THRESHOLD_WINDOW_LIST ausgewählt wird, der der größte unter den in der Liste THRESHOLD_WINDOW_LIST gespeicherten ist, und der Maximalwert FACTOR_MAX2 des Parameters FACTOR, der als doppelt so groß wie die Amplitude der vorherigen R-Welle im QRS-Komplex angesehen wird, in das Modul AMPLITUDE MEMORY, anstelle der zuvor für den Wert FACTOR_MAX1 des Parameters FACTOR erkannten R-Welle wird, und dann der Maximalwert FACTOR_MAX2 des Parameters FACTOR in das Ausgabespeichermodul OUTPUT MEMORY kopiert wird,

gleichzeitig ein Zeitpunkt des Auftretens des Maximalwerts FACTOR_MAX2 des Parameters FACTOR im Modul TIMESTAMP MEMORY gespeichert wird, der auch als Zeitpunkt des Auftretens der vorherigen R-Welle im QRS-Komplex erkannt wird, anstelle der zuvor für den Wert FACTOR_MAX1 des Parameters FACTOR erkannten R-Welle, und dann der Zeitpunkt des Auftretens des Maximalwerts FACTOR_MAX2 des Parameters FACTOR in das Ausgabespeichermodul OUTPUT MEMORY kopiert wird,

als nächstes der Summenwert der Werte der Parameter FACTOR_MAX1, die bisher erhalten wurden, mittels des Moduls TH FACTOR MODULE aktualisiert wird, indem der aktuelle Wert FACTOR_MAX1 zum vorherigen Summenwert addiert wird und ein Zähler der durchgeführten Algorithmus-Arbeitszyklen aktualisiert wird und auf dieser Grundlage ein arithmetischer Mittelwert der bisher innerhalb von Zeitfenstern gefundenen Werte FACTOR berechnet wird,

und dann dem Parameter TH_FACTOR der Wert aus dem Paar MIN_TH_FACTOR und NEW_TH_FACTOR zugewiesen wird, der nicht kleiner ist als der zweite Wert im Paar MIN_TH_FACTOR und NEW_TH_FACTOR, wobei NEW_TH_FACTOR das Produkt eines Parameters EXPECTED_TH_FACTOR und eines gewichteten Mittelwerts mit den Gewichten (1 - TH_FACTOR_LAST_PEAK_COEF) und TH_FACTOR_LAST_PEAK_COEF des arithmetischen Mittelwerts der Werte des Parameters FACTOR für die bisher gefundenen R-Wellen im QRS-Komplex und des Werts des Parameters FACTOR_MAX1 für die zuletzt gefundene R-Welle ist,

als nächstes der Summenwert der bisher erhaltenen Maximalwerte des Signals Y_PREPROCESSED aktualisiert wird mittels des Moduls TH MODULE durch Addition des aktuellen Maximalwerts Y_PREPROCESSED zum vorherigen Summenwert und Aktualisierung des Zählers der durchgeführten Algorithmus-Arbeitszyklen und auf dieser Grundlage Berechnung eines arithmetischen Mittels der Werte Y_PREPROCESSED, die bisher innerhalb von Zeitfenstern gefunden wurden,

als nächstes dem Parameter TH_PREPROCESSED ein neuer Wert zugewiesen wird, der ein gewichteter Mittelwert mit den Gewichten (1-TH_PREPROCESSED_LAST_PEAK_COEF) und TH_PREPROCESSED_LAST_PEAK_COEF des arithmetischen Mittels der Maximalwerte des Signals Y_PREPROCESSED ist, die bisher innerhalb von Zeitfenstern zur Erkennung von R-Wellen im QRS-Komplex gefunden wurden, und des Maximalwerts des Signals Y_PREPROCESSED für das aktuelle Zeitfenster SEARCHING_WINDOW, in dem die letzte R-Welle identifiziert wurde,

als nächstes mittels eines Moduls REFRACTORY WINDOW PULSE GENERATOR eine Messung eines Zeitfensters REFRACTORY_WINDOW gestartet wird, dessen Ende nach der Zeit REFRACTORY_LEN seit dem Zeitpunkt der Erfassung der letzten R-Welle eintritt,

als nächstes während REFRACTORY_WINDOW alle gespeicherten Elemente in den Listen THRESHOLD_WINDOW_LIST und SEARCHING_WINDOW_LIST aus dem Modul FACTOR MEMORY entfernt werden, um das Modul für den nächsten Arbeitszyklus vorzubereiten,

nach Abschluss der Messung des Zeitfensters REFRACTORY_WINDOW die Überwachung des am Ausgang des Moduls PREPROCESSING MODULE generierten Signals Y_PREPROCESSED mittels des Komparators COMPARATOR wieder aufgenommen wird und der Zyklus dann das gewünschte Vielfache wiederholt wird.

2. Das Verfahren nach Anspruch 1, **dadurch gekennzeichnet, dass** die lokalen Extrema des EKG-Signals mittels des Moduls EXTREMA MODULE so gesucht werden, dass

- nach dem Empfangen der betrachteten Probe des EKG-Signals mittels des Messmoduls ECG MODULE und dem Speichern dieser Probe des EKG-Signals mittels des Moduls BUF ein Maximalwert LOCAL_EXT_MAX_LEFT und ein Minimalwert LOCAL_EXT_MIN_LEFT mittels des EXTREMA MODULE für einen Satz von vorbestimmten Zahlen LOCAL_EXT_LEN von Proben des EKG-Signals, die im Puffer BUF gespeichert sind und die der betrachteten Probe des EKG-Signals unmittelbar vorangehen, bestimmt werden, und dann der Maximalwert LOCAL_EXT_MAX_LEFT und der Minimalwert LOCAL_EXT_MIN_LEFT mittels des Moduls EXTREMA MODULE gespeichert werden, und auch

- nach dem Empfangen mittels des Messmoduls ECG_MODULE und dem Sammeln dieser Probe des EKG-Signals im Puffer BUF der vorbestimmten Anzahl LOCAL_EXT_LEN von Proben des EKG-Signals, die unmittelbar darauf folgen der betrachteten Probe des EKG-Signals ein Maximalwert LOCAL_EXT_MAX_RIGHT und ein Minimalwert LOCAL_EXT_MIN_RIGHT mittels des Moduls EXTREMA MODULE für einen Satz von vorgegebenen Zahlen LOCAL_EXT_LEN von Proben des EKG-Signals und der betrachteten Probe des EKG-Signals bestimmt werden, und dann dieser Maximalwert LOCAL_EXT_MAX_RIGHT und Minimalwert LOCAL_EXT_MIN_RIGHT mittels des Moduls EXTREMA MODULE gespeichert werden,

als nächstes der zuvor bestimmte Maximalwert LOCAL_EXT_MAX_LEFT und der zuvor bestimmte Minimalwert LOCAL_EXT_MIN_LEFT mittels des Moduls EXTREMA MODULE nacheinander verglichen werden, für die vorgegebene Anzahl LOCAL_EXT_LEN von Proben des EKG-Signals, die der betrachteten Probe des EKG-Signals unmittelbar vorangehen und im Puffer BUF gespeichert sind, mit dem Wert Y der betrachteten Probe des EKG-Signals,

und auch der zuvor bestimmte Maximalwert LOCAL_EXT_MAX_ RIGHT und der zuvor bestimmte LOCAL_EXT_MIN_RIGHT nacheinander verglichen werden durch mittels des Moduls EXTREMA MODULE für die vorgegebene Anzahl LOCAL_EXT_LEN von Proben des EKG-Signals, die unmittelbar auf die betrachtete Probe des EKG-Signals folgen, mit dem Wert Y der betrachteten Probe des EKG-Signals verglichen werden,

und wenn das Ergebnis der Vergleiche für mindestens einen der Maximalwerte LOCAL_EXT_MAX_LEFT und LOCAL_EXT_MAX_ RIGHT oder der Minimalwerte LOCAL_EXT_MIN_LEFT und LOCAL_EXT_MIN_RIGHT, die zuvor für die Gruppen von Proben erhalten wurden, die der betrachteten Probe des EKG-Signals unmittelbar vorangehen und unmittelbar folgen, Gleichheit mit dem Wert Y der betrachteten Probe des EKG-Signals zeigt, dann die betrachtete Probe des EKG-Signals als lokales Extrem erkannt wird.

3. Das Verfahren gemäß Anspruch 1, **dadurch gekennzeichnet, dass** der Wert des Parameters FACTOR, der angibt, wie deutlich sich das lokale Extremum der betrachteten Probe des EKG-Signals von den benachbarten Proben des EKG-Signals unterscheidet, mittels des Moduls FACTOR MODULE so bestimmt wird, dass

ein Minimalwert MIN_LEFT und ein Maximalwert MAX_LEFT von Proben des EKG-Signals für eine vorgegebene Anzahl MAX_WIDTH von Proben des EKG-Signals bestimmt werden, die der betrachteten Probe unmittelbar vorausgehen, und im Puffer BUF gespeichert werden, und dann dieser Maximalwert MAX_LEFT und dieser Minimalwert MIN_LEFT mittels des Moduls FACTOR MODULE für die vorgegebene Anzahl MAX_WIDTH von Proben des EKG-Signals gespeichert werden, die der betrachteten Probe unmittelbar vorausgehen, und außerdem

- nach dem Empfangen mittels des Messmoduls ECG MODULE und Sammeln im Puffer BUF der vorgegebenen Anzahl MAX_WIDTH von Proben des EKG-Signals, die unmittelbar auf die betrachtete Probe des EKG-Signals folgen, ein Maximalwert MAX_RIGHT und ein Minimalwert MIN_RIGHT mittels des Moduls FACTOR MODULE für die vorgegebene Anzahl MAX_WIDTH von Proben des EKG-Signals bestimmt wird, und dann der Maximalwert MAX_RIGHT und der Minimalwert MIN_RIGHT mittels des Moduls FACTOR MODULE für die vorgegebene Anzahl MAX_WIDTH von Proben des EKG-Signals gespeichert werden, die unmittelbar auf die betrachtete Signalprobe folgen,

und der Wert des Parameters FACTOR bestimmt wird, indem als Parameter FACTOR der Wert desjenigen aus dem Paar der Parameter FACTOR_MAX und FACTOR_MIN genommen wird, der nicht kleiner als der zweite ist, wobei die Werte der Parameter FACTOR_MAX und FACTOR_MIN gemäß den Formeln bestimmt werden:

$$FACTOR\_MAX = (Y - MIN\_LEFT) + (Y - MIN\_RIGHT)$$

$$FACTOR\_MIN = (MAX\_LEFT - Y) + (MAX\_RIGHT - Y)$$

wobei Y der Wert der betrachteten Probe des EKG-Signals ist, für die der Faktor FACTOR bestimmt ist.

**4.** Ein Speichermedium, das computerausführbare Anweisungen zur Implementierung des Verfahrens gemäß den Ansprüchen 1-3 enthält.

**Revendications**

**1.** Procédé de détection de complexe QRS dans un signal d'électrocardiogramme (ECG) consistant à recevoir le signal ECG obtenu au moyen d'un module de mesure ECG MODULE surveillant l'activité électrique cardiaque d'un patient, dans lequel le signal ECG est représenté sous forme d'échantillons fournis par le module de mesure ECG MODULE à des intervalles de temps égaux mesurés par une horloge et stockés successivement dans un tampon BUF au moyen d'un signal CLK généré dans le module de mesure ECG MODULE, puis en ce que

a) au moyen d'un module TH MODULE on fixe une valeur initiale d'un seuil TH_PREPROCESSED, puis
b) on surveille au moyen d'un comparateur COMPARATOR un signal Y_PREPROCESSED généré à une sortie d'un module PREPROCESSING MODULE, lequel signal est obtenu en générant au moyen du module PREPROCESSING MODULE un signal *Y0* étant la différence entre une valeur *Y* de l'échantillon considéré du signal ECG et une valeur d'échantillon du signal ECG préalablement stockée dans le tampon BUF, qui a été reçue un nombre prédéterminé *Nd* des échantillons précédents,
et en déterminant au moyen du module PREPROCESSING MODULE un signal *Y1* qui est la somme de l'échantillon considéré du signal *Y0* et une valeur d'un nombre prédéterminé *N-1* d'échantillons du signal *Y0* précédant immédiatement l'échantillon considéré du signal *Y0,* et en divisant cette somme par un nombre *N* et en élevant au carré ce nombre obtenu,
c) au moyen du module TH MODULE, la valeur du seuil TH_PREPROCESSED est systématiquement diminuée à chaque intervalle d'échantillonnage du signal ECG à partir de sa valeur initiale préalablement déterminée au moyen du module TH MODULE en multipliant la valeur de seuil courante TH_PREPROCESSED, dans les instants de temps de réception d'échantillons successifs du signal ECG, par une valeur constante prédéterminée et inférieure à une TH_REDUCING_FACTOR et ces opérations sont répétées le nombre de fois requis jusqu'à ce qu'un instant de temps d'atteinte du seuil TH_PREPROCESSED par le signal Y_PREPROCESSED soit détecté au moyen du comparateur COMPARATOR,
d) lorsque l'instant de temps d'atteinte de la valeur courante du seuil TH_PREPROCESSED par le signal Y_PREPROCESSED est détecté, une fenêtre temporelle SEARCHING_WINDOW est commencée à être mesurée au moyen d'un générateur d'impulsions SEARCHING WINDOW PULSE GENERATOR pour rechercher l'onde R dans le complexe QRS du signal ECG,
e) ensuite, pendant la fenêtre temporelle SEARCHING_WINDOW une valeur maximale du signal Y_PREPROCESSED est détectée au moyen d'un module de détection PEAK DETECTOR et cette valeur est stockée dans un module PEAK AMPLITUDE MEMORY,
**caractérisé en ce que**

à l'étape a) une valeur initiale pour un seuil TH_FACTOR est définie au moyen d'un module TH FACTOR MODULE,

à l'étape c) des extrêmes locaux du signal ECG sont recherchés au moyen d'un module EXTREMA MODULE,

après avoir détecté au moyen du module EXTREMA MODULE que l'échantillon considéré du signal ECG est l'extrême local du signal ECG, une valeur d'un paramètre FACTOR est déterminée pour cet échantillon du signal ECG au moyen d'un module FACTOR MODULE, lequel paramètre spécifie à quel point l'extrême local considéré de l'échantillon du signal ECG est distinct des échantillons voisins du signal ECG, et à cet effet le paramètre FACTOR se voit attribuer une valeur d'autant plus grande que l'amplitude de l'extrême local considéré représenté par l'échantillon considéré du signal ECG est grande que les valeurs des échantillons voisins du signal ECG,

ensuite, après avoir déterminé la valeur du paramètre FACTOR pour l'échantillon considéré du signal ECG qui est l'extrême local du signal ECG, la valeur déterminée du paramètre FACTOR est comparée au seuil TH_FACTOR précédemment déterminé au moyen du module TH FACTOR MODULE, et si la valeur du paramètre FACTOR est supérieure au seuil TH_FACTOR défini, la valeur du paramètre FACTOR et l'instant de réception de l'échantillon considéré du signal ECG précédemment enregistré au moyen du module EXTREMA MODULE, pour lequel instant le paramètre FACTOR a été déterminé, sont stockés dans une liste THRESHOLD_WINDOW_LIST dans une mémoire d'un module FACTOR MEMORY,

où ces opérations sont répétées le nombre de fois requis jusqu'à ce qu'un instant de temps d'atteinte du seuil TH_PREPROCESSED par le signal Y_PREPROCESSED soit détecté au moyen du comparateur COMPARATOR, dont la valeur est systématiquement diminuée à chaque intervalle d'échantillonnage du signal ECG par multiplication successive de la valeur du seuil TH_PREPROCESSED, dans le temps instants de réception d'échantillons successifs du signal ECG, par la valeur prédéterminée et inférieure à une constante TH_REDUCING_FACTOR,

où à l'étape d) la fenêtre temporelle SEARCHING_WINDOW a une longueur minimale déterminée SEARCHING_WINDOW_LEN_MIN,

à l'étape e) pendant la fenêtre temporelle SEARCHING_WINDOW les extrêmes locaux du signal ECG sont déterminés simultanément au moyen du module EXTREMA MODULE,

ensuite, pour chacun des échantillons reconnus comme extrême local, on détermine, en déterminant une valeur du paramètre FACTOR au moyen du module FACTOR MODULE, à quel point l'extrême local de l'échantillon considéré du signal ECG est distinct des échantillons voisins,

pour chaque extrême local détecté, la valeur du paramètre FACTOR et un instant temporel de réception de l'échantillon considéré du signal ECG précédemment enregistré au moyen du module EXTREMA MODULE, pour lequel instant temporel le paramètre FACTOR a été déterminé, sont stockés dans une liste SEARCHING_WINDOW_LIST dans le module de mémoire FACTOR MEMORY,

après une durée minimale définie SEARCHING_WINDOW_LEN_MIN de la fenêtre temporelle SEARCHING_WINDOW la mesure de la fenêtre temporelle SEARCHING_WINDOW est terminée par le générateur d'impulsions SEARCHING WINDOW PULSE GENERATOR, à condition que la valeur maximale du signal Y_PREPROCESSED enregistrée pendant la fenêtre temporelle SEARCHING_WINDOW et réduite de la valeur actuelle du signal Y_PREPROCESSED atteigne un seuil prédéterminé MIN_DIFF, ce qui est détecté par un module PULSE EXTENSION,

après la fin de la fenêtre temporelle SEARCHING_WINDOW une telle valeur FACTOR_MAX1 du paramètre FACTOR est sélectionnée dans la liste SEARCHING_WINDOW_LIST au moyen d'un module FACTOR SELECTOR, qui est la plus grande parmi celles stockées dans la liste SEARCHING_WINDOW_LIST et la valeur maximale FACTOR_MAX1 du paramètre FACTOR considérée comme le double de l'amplitude de l'onde R dans le complexe QRS est stockée dans un module AMPLITUDE MEMORY, puis la valeur maximale la valeur FACTOR_MAX1 du paramètre FACTOR est copiée dans un module de mémoire de sortie OUTPUT MEMORY,

en même temps, un instant d'apparition de la valeur maximale FACTOR_MAX1 du paramètre FACTOR est stocké dans un module TIMESTAMP MEMORY, qui est également reconnu comme l'instant d'apparition de l'onde R dans le complexe QRS, puis l'instant d'apparition de la valeur maximale FACTOR_MAX1 du paramètre FACTOR est copié dans le module de mémoire de sortie OUTPUT MEMORY,

ensuite, à partir de la liste THRESHOLD_WINDOW_LIST dans le module FACTOR MEMORY, les valeurs du paramètre FACTOR stockées dans cette liste sont supprimées au moyen du module FACTOR SELECTOR, dont l'instant d'apparition précède de moins d'une période de temps définie R-TO-R_MIN depuis l'instant d'apparition de l'onde R dans le complexe QRS stocké dans le module de mémoire de sortie OUTPUT MEMORY,

ensuite, à condition que le nombre d'éléments restants dans la liste THRESHOLD_WINDOW_LIST ne soit pas supérieur à une constante définie MAX_ABOVE_TH, que la valeur FACTOR_MAX2 du paramètre FACTOR soit sélectionnée dans la liste THRESHOLD_WINDOW_LIST au moyen d'un module FACTOR SELECTOR, qui est la plus grande parmi celles stockées dans la liste THRESHOLD_WINDOW_LIST et la valeur maximale FACTOR_MAX2 du paramètre FACTOR considérée comme le double de l'amplitude de l'onde R précédente dans le complexe QRS est stockée dans le module AMPLITUDE MEMORY, au lieu de l'onde R précédemment détectée pour la valeur FACTOR_MAX1 du paramètre FACTOR, puis la valeur maximale FACTOR_MAX2 du paramètre FACTOR est copiée dans le module de mémoire de sortie OUTPUT MEMORY,

en même temps un instant de temps d'apparition de la valeur maximale FACTOR_MAX2 du paramètre FACTOR est stocké dans le module TIMESTAMP MEMORY, qui est également reconnu comme l'instant de temps d'apparition de l'onde R précédente dans le complexe QRS, au lieu de l'onde R précédemment détectée pour la valeur FACTOR_MAX1 du paramètre FACTOR, puis l'instant de temps d'apparition de la valeur maximale FACTOR_MAX2 du paramètre FACTOR est copié dans le module de mémoire de sortie OUTPUT MEMORY,

ensuite, la valeur de somme des valeurs des paramètres FACTOR_MAX1 obtenues jusqu'à présent est mise à jour au moyen du module TH FACTOR MODULE en ajoutant la valeur actuelle FACTOR_MAX1 à la valeur de somme précédente et un compteur de cycles de travail d'algorithme effectués est mis à jour et sur cette base une moyenne arithmétique des valeurs FACTOR trouvé jusqu'à présent dans les fenêtres temporelles est calculé,

puis le paramètre TH_FACTOR se voit attribuer cette valeur parmi la paire MIN_TH_FACTOR et NEW_TH_FACTOR, qui n'est pas inférieure à la deuxième valeur de la paire MIN_TH_FACTOR et NEW_TH_FACTOR, où NEW_TH_FACTOR est le produit d'un paramètre EXPECTED_TH_FACTOR et d'une moyenne pondérée avec des poids, respectivement, (1-TH_FACTOR_LAST_PEAK_COEF) et TH_FACTOR_LAST_PEAK_COEF de la moyenne arithmétique des valeurs du paramètre FACTOR pour les ondes R dans le complexe QRS trouvées jusqu'à présent et la valeur du paramètre FACTOR_MAX1 pour la dernière onde R trouvée,

ensuite, la valeur de somme des valeurs maximales du signal Y_PREPROCESSED obtenues jusqu'à présent est mise à jour au moyen du module TH MODULE en ajoutant la valeur maximale actuelle Y_PREPROCESSED à la valeur de somme précédente et au compteur de les cycles de travail de l'algorithme exécutés sont mis à jour et sur cette base, une moyenne arithmétique des valeurs Y_PREPROCESSED trouvées jusqu'à présent dans les fenêtres temporelles est calculée,

ensuite, une nouvelle valeur est attribuée au paramètre TH_PREPROCESSED, qui est une moyenne pondérée avec des poids, respectivement, (1-TH_PREPROCESSED_LAST_PEAK_COEF) et TH_PREPROCESSED_LAST_PEAK_COEF de la moyenne arithmétique des valeurs maximales du signal Y_PREPROCESSED trouvées jusqu'à présent dans les fenêtres temporelles de détection des ondes R dans le complexe QRS et de la valeur maximale du signal Y_PREPROCESSED pour la fenêtre temporelle actuelle SEARCHING_WINDOW, dans laquelle la dernière onde R a été identifiée,

ensuite, au moyen d'un module REFRACTORY WINDOW PULSE GENERATOR, une mesure d'une fenêtre temporelle REFRACTORY_WINDOW est lancée, dont la fin se produit après le temps REFRACTORY_LEN depuis l'instant de détection la dernière onde R,

ensuite, pendant REFRACTORY_WINDOW tous les éléments stockés dans les listes THRESHOLD_WINDOW_LIST et SEARCHING_WINDOW_LIST sont supprimés du module FACTOR MEMORY afin de préparer le module pour le prochain cycle de travail,

après avoir terminé la mesure de la fenêtre temporelle REFRACTORY _WINDOW, la surveillance du signal Y_PREPROCESSED généré à la sortie du module PREPROCESSING MODULE au moyen du comparateur COMPARATOR est reprise, puis le cycle est répété le nombre de fois souhaité.

2. Procédé selon la revendication 1, **caractérisé en ce que** les extrêmes locaux du signal ECG sont recherchés au moyen du module EXTREMA MODULE de telle manière que

- après réception de l'échantillon considéré du signal ECG au moyen du module de mesure ECG MODULE et stockage de cet échantillon du signal ECG au moyen du module BUF, une valeur maximale LOCAL_EXT_MAX_LEFT et une valeur minimale LOCAL_EXT_MIN_LEFT sont déterminées au moyen du EXTREMA MODULE pour un ensemble de nombre prédéterminé LOCAL_EXT_LEN d'échantillons du signal ECG stockés dans la mémoire tampon BUF qui précèdent immédiatement l'échantillon considéré et de l'échantillon considéré du signal ECG, puis la valeur maximale LOCAL_EXT_MAX_LEFT et la valeur minimale LOCAL_EXT_MIN_LEFT sont stockées au moyen du module EXTREMA MODULE, et également

- après réception au moyen du module de mesure ECG_MODULE et collecte dans la mémoire tampon BUF le nombre prédéterminé LOCAL_EXT_LEN des échantillons du signal ECG qui suivent immédiatement l'échantillon considéré du signal ECG, une valeur maximale LOCAL_EXT_MAX_RIGHT et une valeur minimale LOCAL_EXT_MIN_RIGHT sont déterminées au moyen du module EXTREMA MODULE pour un ensemble de nombre prédéterminé LOCAL_EXT_LEN d'échantillons du signal ECG et de l'échantillon considéré du signal ECG, puis ces valeur maximale LOCAL_EXT_MAX_RIGHT et valeur minimale LOCAL_EXT_MIN_RIGHT sont mémorisées au moyen du module EXTREMA MODULE,

ensuite, la valeur maximale LOCAL_EXT_MAX_LEFT et la valeur minimale LOCAL_EXT_MIN_LEFT déterminées précédemment sont comparées successivement au moyen du module EXTREMA MODULE, pour le nombre prédéterminé LOCAL_EXT_LEN d'échantillons du signal ECG précédant immédiatement l'échantillon considéré du signal ECG et mémorisées dans le tampon BUF, avec la valeur Y de l'échantillon considéré du signal ECG,

ainsi que la valeur maximale LOCAL_EXT_MAX_ déterminée précédemment RIGHT et le LOCAL_EXT_MIN_RIGHT précédemment déterminé sont comparés successivement au moyen du module EXTREMA MODULE, pour le nombre prédéterminé LOCAL_EXT_LEN d'échantillons du signal ECG qui suivent immédiatement l'échantillon considéré du signal ECG, avec la valeur Y de l'échantillon considéré du signal ECG, et si le résultat des comparaisons montre une égalité avec la valeur Y de l'échantillon considéré du signal ECG pour au moins une des valeurs maximales LOCAL_EXT_MAX_LEFT et LOCAL_EXT_MAX_RIGHT ou des valeurs minimales LOCAL_EXT_MIN_LEFT et LOCAL_EXT_MIN_RIGHT obtenues précédemment pour les groupes d'échantillons précédant immédiatement et suivant immédiatement l'échantillon considéré du signal ECG, alors l'échantillon considéré du signal ECG est reconnu comme un extrême local.

3. Procédé selon la revendication 1, **caractérisé en ce que** la valeur du paramètre FACTOR, qui spécifie à quel point l'extrême local de l'échantillon considéré du signal ECG est distinct des échantillons voisins du signal ECG, est déterminée au moyen du module FACTOR MODULE de telle manière que

une valeur minimale MIN_LEFT et une valeur maximale MAX_LEFT d'échantillons du signal ECG sont déterminées pour un nombre prédéterminé MAX_WIDTH d'échantillons du signal ECG qui précèdent immédiatement l'échantillon considéré et sont stockées dans la mémoire tampon BUF, puis, cette valeur maximale MAX_LEFT et cette valeur minimale MIN_LEFT sont stockées au moyen du module FACTOR MODULE pour le nombre prédéterminé MAX_WIDTH d'échantillons du signal ECG qui précèdent immédiatement l'échantillon considéré, et également

- après réception au moyen du module de mesure ECG MODULE et collecte dans la mémoire tampon BUF du nombre prédéterminé MAX_WIDTH d'échantillons du signal ECG qui suivent immédiatement l'échantillon considéré du signal ECG, une valeur maximale MAX_RIGHT et une valeur minimale MIN_RIGHT sont déterminées par au moyen du module FACTOR MODULE pour le nombre prédéterminé MAX_WIDTH d'échantillons du signal ECG, puis la valeur maximale MAX_RIGHT et la valeur minimale MIN_RIGHT sont mémorisées au moyen du module FACTOR MODULE pour le nombre prédéterminé MAX_WIDTH d'échantillons du signal ECG qui suivent immédiatement l'échantillon de signal considéré,

et la valeur du paramètre FACTOR est déterminée en prenant comme paramètre FACTOR la valeur de celui parmi la paire de paramètres FACTOR_MAX et FACTOR_MIN, qui n'est pas inférieure à la seconde, où les valeurs des paramètres FACTOR_MAX et FACTOR_MIN sont déterminées selon les formules :

$$FACTOR\_MAX = (Y - MIN\_LEFT) + (Y - MIN\_RIGHT)$$

$$FACTOR\_MIN = (MAX\_LEFT - Y) + (MAX\_RIGHT - Y)$$

où $Y$ est la valeur de l'échantillon considéré du signal ECG, pour lequel le facteur FACTOR est déterminé.

4. Support de stockage comprenant des instructions exécutables par ordinateur pour mettre en œuvre le procédé selon les revendications 1 à 3.

**Fig. 1**

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GUTIÉRREZ-RIVAS, RAQUEL et al.** Novel real-time low-complexity QRS complex detector based on adaptive thresholding.. *IEEE Sensors Journal*, 2015, vol. 15 (10), 6036-6043